# EUROPEAN PATENT APPLICATION

(11) **EP 4 835 067 A1**
(43) Date of publication of application: **04.11.2026**
(21) Application number: 24913296.0
(22) Date of filing: 29.09.2024
(51) Int. Cl.: C12N 9/26, C12N 15/85, A61K 9/00, A61K 47/42

(54) **HYALURONIDASE HYAL1 VARIANT**

(30) Priority: 28.12.2023 KR 20230194917
(71) Applicant: Odysgen Inc., Daegu 41256 (KR)
(72) Inventor: CHOI, Mal-Gi, Daegu 41256 (KR); LEE, Chang Woo, Daegu 42088 (KR)
(74) Representative: dompatent
(86) International application number: PCT/KR2024/014816
(87) International publication number: WO 2025/143455

(57) **Abstract**

The present invention provides a Hyal1 variant derived from wild-type human hyaluronidase Hyal1 including glutamic acid 131 as a catalytic amino acid, wherein one or more amino acids adjacent to the catalytic amino acid glutamic acid 131 in the primary structure or tertiary structure are substituted with an acidic, polar, or basic amino acid. The present invention also provides a method for producing the Hyal1 variant, a nucleic acid, an expression vector, and a host cell for producing the Hyal1 variant, as well as formulations or uses thereof. The Hyal1 variant of the present invention is capable of effectively degrading hyaluronic acid not only under acidic pH conditions but also under neutral pH conditions, thereby providing high utility.

## Description

### [Technical Field]

The present invention relates to a technology for altering the active pH range and enzymatic activity of human hyaluronidase, and specifically, to a hyaluronidase Hyal1 variant that hydrolyzes hyaluronic acid over an acidic-to-neutral pH range and exhibits enhanced enzymatic activity at an acidic pH.

### [Background Art]

Hyaluronidase is an enzyme that hydrolyzes hyaluronic acid, and hyaluronidases present in humans include Hyal1, Hyal2, Hyal3, Hyal4, and PH20.

Hyaluronidase PH20 hydrolyzes hyaluronic acid in the extracellular matrix, and due to this property, it can be used in clinical subcutaneous injection formulations of antibody therapeutics to enhance subcutaneous drug delivery. However, the hydrolysis of hyaluronic acid currently relies on a single enzyme, PH20. That is, among hyaluronidases other than PH20, Hyal1 primarily exhibits catalytic activity under acidic conditions of pH 3 to 4, and thus its utility is limited. For example, when Hyal1 is applied to a subcutaneous site with a neutral pH of 7.0 to 7.5, it is difficult to expect effective degradation of hyaluronic acid. In addition, since wild-type Hyal2, Hyal3, and Hyal4 also exhibit weak activity in a neutral pH range, they are not used in practice.

In addition, enzyme replacement therapy or gene therapy is required for the treatment of Hyal1 deficiency; however, little research has been conducted on increasing the enzymatic activity of Hyal1 at an acidic pH.

Accordingly, the present inventors conducted research to improve the utility of wild-type Hyal1, and as a result, completed the present invention.

### [Summary of Invention]

### [Problems to be Solved by Invention]

An object of the present invention is to provide a Hyal1 variant capable of effectively hydrolyzing hyaluronic acid in an acidic-to-neutral pH range and a Hyal1 variant having higher enzymatic activity than wild-type Hyal1 at an acidic pH.

### [Means for Solving Problems]

A human hyaluronidase Hyal1 variant of the present invention has the acidic amino acids Asp129 and Glu131 at the active site. Glu131 functions as a general acid-base catalyst during catalysis, and Asp129 stabilizes a substrate during catalysis.

The present invention provides a Hyal1 variant in which an amino acid adjacent to the catalytic amino acid Glu131 in the primary structure or tertiary structure of Hyal1 is substituted with an acidic or basic amino acid to enable Hyal1 to exhibit catalytic activity at neutral pH.

The amino acids adjacent to the catalytic amino acid Glu131 in the primary structure or tertiary structure of Hyal1 may be Ala132, amino acids located in a loop composed of amino acids Ser76 to Leu98, or an amino acid adjacent to an acidic or polar amino acid that may influence the activity of the catalytic amino acid in the tertiary structure of Hyal1.

Ala132 may be substituted with an acidic, polar, or basic amino acid. In this case, the acidic amino acid may be Asp or Glu, the polar amino acid may be Ser, Thr, Asn, Gln, or Tyr, and the basic amino acid may be His.

In a loop composed of amino acids Ser76 to Leu98, the amino acids adjacent to the catalytic amino acid Glu131 in the tertiary structure are Ser76, Ser77, Thr86, Pro87, or Thr88, and these amino acids are substituted with Asp or Glu.

In addition, any one of the amino acids adjacent to the catalytic amino acid Glu131 in the tertiary structure of Hyal1 is substituted with a basic amino acid. In this case, the substituted basic amino acid may form an ionic bond with an acidic or polar amino acid adjacent to the catalytic amino acid Glu131. For example, in the tertiary structure of Hyal1, amino acids adjacent to Glu131 that may affect the activity of the catalytic amino acid include the acidic amino acid Asp206 and the polar amino acid Tyr210. When Phe139 adjacent to Tyr210 is substituted with the basic amino acid Arg, an ionic bond is formed between Arg139 and Tyr210. Likewise, when Tyr210 adjacent to Asp206 is substituted with the basic amino acid His, an ionic bond is formed between Asp206 and His210. As a result, the Hyal1 variant of the present invention may hydrolyze hyaluronic acid even at neutral pH.

The present invention also provides a Hyal1 variant in which an amino acid adjacent to the catalytic amino acid Glu131 in the primary structure or tertiary structure of Hyal1 is substituted with an acidic amino acid, or Ala132 is substituted with an amino acid larger than Ala, thereby increasing the enzymatic activity of Hyal1 at an acidic pH.

The amino acids adjacent to the catalytic amino acid Glu131 in a loop composed of amino acids Ser76 to Leu98 are Ser76, Ser77, Thr86, Pro87, Thr88, or Gly89.

In addition, amino acids larger than Ala132 include all acidic amino acids, polar amino acids, basic amino acids, and hydrophobic amino acids except for Gly, which is smaller than Ala.

In addition, the present invention provides a nucleic acid encoding the hyaluronidase Hyal1 variant.

Further, the present invention provides a recombinant expression vector including the nucleic acid.

Further, the present invention provides a host cell transformed with the recombinant expression vector.

Further, the present invention provides a method for producing a hyaluronidase Hyal1 variant, including culturing the host cell.

Further, the present invention provides a hyaluronic acid degrading agent including the hyaluronidase Hyal1 variant.

Further, the present invention provides a drug delivery agent including the hyaluronidase Hyal1 variant.

Further, the present invention provides a formulation for intravenous injection including the hyaluronidase Hyal1 variant.

Further, the present invention provides a formulation for subcutaneous administration including the hyaluronidase Hyal1 variant.

Further, the present invention provides an ophthalmic formulation including the hyaluronidase Hyal1 variant.

### [Advantageous effects]

The hyaluronidase Hyal1 variant of the present invention may effectively degrade hyaluronic acid in an acidic-to-neutral pH range and thus has improved utility.

That is, the hyaluronidase of the present invention is capable of degrading hyaluronic acid at neutral pH, thereby enabling drug delivery, and may also be used in various formulations or applications, such as a subcutaneous formulation, intravenous formulations, and ophthalmic formulations.

In addition, since the Hyal1 variant of the present invention exhibits higher enzymatic activity than wild-type Hyal1 at an acidic pH, the variant may be developed as a therapeutic agent for Hyal1 deficiency through enzyme replacement therapy or gene therapy.

Furthermore, the hyaluronidase of the present invention may be effectively produced using the nucleic acid, expression vector, host cell, and method of the present invention.

### [Brief Description of Drawings]

FIG. 1(A) illustrates the results of an experiment examining whether wild-type Hyal1 and Ala132 variants exhibit enzymatic activity depending on pH.
FIG. 1(B) illustrates the results of a Western blot experiment confirming expression of wild-type Hyal1 and Ala132 variants in CHO-K1 cells.
FIG. 1(C) illustrates the results of an experiment comparing the enzymatic activity of wild-type Hyal1 and the A132G variant.
FIG. 2(A) illustrates the results of an experiment examining whether wild-type Hyal1 and Ser76 variants exhibit enzymatic activity depending on pH.
FIG. 2(B) illustrates the results of an experiment examining whether wild-type Hyal1 and Ser77 variants exhibit enzymatic activity depending on pH.
FIG. 2(C) illustrates the results of an experiment examining whether wild-type Hyal1 and Tyr85, Thr86, Pro87, Thr88, or Gly89 variants exhibit enzymatic activity. The A132E variant was used as a positive control in FIGS. 2(A) and 2(C).
FIG. 3(A) illustrates the results of an experiment examining whether variants having two or more substitutions among Ser77, Thr86, and Ala132 exhibit enzymatic activity depending on pH.
FIG. 3(B) illustrates the results of a Western blot experiment confirming the expression of variants having two or more substitutions among Ser77, Thr86, and Ala132 in CHO-K1 cells.
FIG. 4 illustrates a comparison of the active site structures of Hyal1 and PH20. Acidic amino acids Asp and Glu are commonly present in the active sites of Hyal1 and PH20 (Asp129 and Glu131 in Hyal1, and Asp146 and Glu148 in PH20). The catalytic amino acid is Glu131 in Hyal1 and Glu148 in PH20.
FIG. 5(A) illustrates the results of an experiment examining whether wild-type Hyal1 and Phe139, Tyr210, or Phe139/Ile225 variants exhibit enzymatic activity depending on pH.
FIG. 5(B) illustrates the results of a Western blot experiment confirming the expression of wild-type Hyal1 and Phe139, Tyr210, or Phe139/Ile225 variants in CHO-K1 cells.
FIG. 5(C) illustrates the positions of Phe139, Asp206, Tyr210, and Ile225 adjacent to the catalytic amino acid Glu131 in the tertiary structure of Hyal1.

### [Mode for Carrying out Invention]

Hereinafter, the advantages and features of the present invention, and methods for achieving them, will become apparent with reference to the embodiments described in detail below in conjunction with the accompanying drawings. However, the present invention is not limited to the embodiments set forth below, but may be embodied in various different forms. These embodiments are provided only so that the disclosure of the present invention is complete and to fully convey the scope of the invention to those skilled in the art. The present invention is defined solely by the scope of the claims.

Throughout the specification, the term "and/or" encompasses each of the mentioned components and all combinations of one or more thereof. The terminology used herein is intended for the purpose of describing embodiments and is not intended to limit the present invention. As used herein, the singular form also includes the plural form unless otherwise specifically stated. The terms "comprise" and/or "comprising" as used herein do not exclude the presence or addition of one or more other components and/or steps.

A hyaluronidase Hyal1 variant (hereinafter referred to as a Hyal1 variant) according to an embodiment of the present invention refers to a variant in which, based on wild-type human Hyal1 including Glu131 as a catalytic amino acid, one or more amino acids adjacent to the catalytic amino acid Glu131 in the primary structure or tertiary structure are substituted with an acidic, polar, or basic amino acid. That is, the hyaluronidase Hyal1 variant according to an embodiment of the present invention corresponds to a variant of wild-type Hyal1, and by substituting amino acids adjacent to the catalytic amino acid Glu131 in the primary structure or tertiary structure with acidic, polar, or basic amino acids, hyaluronic acid may be effectively degraded in an acidic-to-neutral pH range, thereby enhancing its utility.

As used herein, the expressions such as "Ala132" or "A132," in which a three-letter or one-letter amino acid name is described together with a number, refer to the amino acid at the corresponding position based on the amino acid sequence of SEQ ID NO: 1. For example, "Ala132" and "A132" represent Ala, which is the amino acid located at position 132 in the amino acid sequence of SEQ ID NO: 1. In addition, the term "variant" of wild-type hyaluronidase as used herein also includes variants in which an amino acid at a specific amino acid position is conservatively substituted. In this case, "conservative substitution" means substitution of one or more amino acids with amino acids having similar biological or biochemical properties that do not cause loss of the biological or biochemical function of the variant. Specifically, a "conservatively substituted variant" may be a variant that has sequence identity of, for example, 80% or more, preferably 90% or more, more preferably 95% or more, and most preferably 99% or more, with a Hyal1 variant consisting of an amino acid sequence selected from the group consisting of amino acid sequences of SEQ ID NO: 2 to SEQ ID NO: 26, and has substantially the same function and/or effect.

The Hyal1 variant according to an embodiment of the present invention corresponds to a hyaluronidase capable of degrading hyaluronic acid over a broad acidic-to-neutral pH range. In the hyaluronidase according to an embodiment of the present invention, the wild-type hyaluronidase is Hyal1, which consists of the amino acid sequence of SEQ ID NO: 1 and the Hyal1 variant may include a substitution of one or more amino acids selected from the group consisting of S76D, S76E, S77D, S77E, Y85D, T86D, T86E, P87D, P87E, G89D, A132D, A132E, A132G, A132H, A132N, A132S, F139R, Y210H, and F139R/I225D. That is, the variant according to an embodiment of the present invention may include a substitution of one or more amino acids selected from the group consisting of S76D, S76E, S77D, S77E, Y85D, T86D, T86E, P87D, P87E, T88D, G89D, A132D, A132E, A132G, A132H, A132N, A132S, F139R, Y210H, and F139R/I225D based on wild-type Hyal1 having the amino acid sequence of SEQ ID NO: 1.

Here, S76D means that the Ser residue at position 76 of SEQ ID NO: 1 is substituted with Asp.

Hyal1 variants are those having mutations in some amino acids of the wild-type Hyal1 sequence, specifically amino acid substitutions, including one or more amino acid substitutions selected from the group consisting of S76D, S76E, S77D, S77E, Y85D, T86D, T86E, P87D, P87E, T88D, G89D, A132D, A132E, A132G, A132H, A132N, A132S, F139R, Y210H, and F139R/I225D. Here, the substitution F139R/I225D means that the amino acid substitutions F139R and I225D are both present.

The amino acid sequence of wild-type Hyal1 is shown in Table 1 below. As shown in the sequence in Table 1, Ala132 is located adjacent to the catalytic amino acid Glu131. A Hyal1 variant in which an amino acid adjacent to the catalytic amino acid is substituted with a specific amino acid appears to exhibit characteristics different from those of the wild type while retaining hyaluronic acid hydrolase activity. That is, as confirmed in the experimental examples, when Ala132 is substituted with Asp or Glu, hyaluronic acid may be hydrolyzed even at neutral pH.

That is, when Ala, which is an amino acid at position 132 of the wild-type Hyal1 amino acid sequence, is substituted with Asp or Glu, hyaluronic acid may be hydrolyzed even at neutral pH. However, Hyal1 variants in which Ala132 is substituted with other similar amino acids did not exhibit such an effect.

Accordingly, it can be seen that, when Ala132 is substituted with Asp or Glu in the Hyal1 variant according to an embodiment of the present invention, hyaluronic acid may be effectively degraded even at neutral pH.

In addition, Ser76 or Ser77 is located adjacent to the catalytic amino acid Glu131 in the tertiary structure of Hyal1, and when Ser76 is substituted with Asp or Ser77 is substituted with Asp or Glu, hyaluronic acid may be hydrolyzed at neutral pH. Specifically, when Ser, which is an amino acid at position 76 of the wild-type Hyal1 amino acid sequence, is substituted with Asp, or when Ser, which is an amino acid at position 77 of the wild-type Hyal1 amino acid sequence, is substituted with Asp or Glu, hyaluronic acid may be hydrolyzed even at neutral pH.

Furthermore, Thr86, Pro87, or Thr88 is located adjacent to the catalytic amino acid Glu131 in the tertiary structure of Hyal1, and when Thr86, Pro87, or Thr88 is substituted with Asp or Glu, hyaluronic acid may also be hydrolyzed at neutral pH. Specifically, when the amino acids at positions 86, 87, 88 of the wild-type Hyal1 amino acid sequence, namely, Thr, Pro, and Thr, are substituted with Asp or Glu, hyaluronic acid may be hydrolyzed even at neutral pH.

Meanwhile, Asp206 and Tyr210 are located adjacent to the catalytic amino acid Glu131 in the tertiary structure of Hyal1. When Phe139, which is adjacent to Tyr210, is substituted with a basic amino acid, or when Tyr210, which is adjacent to Asp206, is substituted with a basic amino acid, hyaluronic acid may be hydrolyzed at neutral pH.

Specifically, when Phe139 is substituted with Arg, the hydroxyl group of Tyr210 may be present in a deprotonated state due to an ionic bond between Arg139 and Tyr210, thereby increasing the pKa value of the catalytic amino acid Glu131. In addition, when both Phe139 and Ile225 are substituted with Arg and Asp, respectively (F139R/I225D), the positive charge of Arg139 may be stabilized by an ionic bond between Arg139 and Asp225, and Arg139 may then form an ionic bond with Tyr210, thereby deprotonating the hydroxyl group of Tyr210.

Meanwhile, when Tyr210 is substituted with His, the pKa value of Asp206 may be decreased due to an ionic bond between Asp206 and His210. As a result, the pKa value of the catalytic amino acid Glu131 may be increased due to electrostatic repulsion between negative charges.

Meanwhile, in an embodiment of the present invention, a Hyal1 variant in which Ser76, Ser77, Thr86, Pro87, Thr88, and Gly89 adjacent to the catalytic amino acid Glu131 are substituted with acidic amino acids Asp or Glu may exhibit increased enzymatic activity at pH 4 compared with wild-type Hyal1, thereby enabling effective hydrolysis of hyaluronic acid.

In addition, Ala132 variants may exhibit increased enzymatic activity at pH 4 compared with wild-type Hyal1 not only by substitution with acidic amino acids but also by substitution with polar amino acids (Asn and Ser) or a basic amino acid (His). In particular, the A132H variant may exhibit enzymatic activity not only at pH 4 but also at pH 6.

The amino acid sequence of wild-type Hyal1 is listed in Table 1, the amino acid sequences of Ala132 variants are listed in Table 2, the amino acid sequences of loop variants are listed in Table 3, the amino acid sequences of variants having one or more combined substitutions of Ala132 and loop variants are listed in Table 4, and the amino acid sequences of Phe139 and Tyr210 variants are listed in Table 5.

**[TABLE 1]**

| | Seque nce No. | Amino acid sequence |
|---|---|---|
| Wild-type human Hyal1 | 1 | MAAHLLPICALFLTLLDMAQGFRGPLLPNRPFTTVWNANTQWC LERHGVDVDVSVFDVVANPGQTFRGPDMTIFYSSQLGTYPYYTP TGEPVFGGLPQNASLIAHLARTFQDILAAIPAPDFSGLAVIDWEA WRPRWAFNWDTKDIYRQRSRALVQAQHPDWPAPQVEAVAQDQ FQGAARAWMAGTLQLGRALRPRGLWGFYGFPDCYNYDFLSPN YTGQCPSGIRAQNDQLGWLWGQSRALYPSIYMPAVLEGTGKSQ MYVQHRVAEAFRVAVAAGDPNLPVLPYVQIFYDTTNHFLPLDEL EHSLGESAAQGAAGVVLWVSWENTRTKESCQAIKEYMDTTLGP FILNVTSGALLCSQALCSGHGRCVRRTSHPKALLLLNPASFSIQLT PGGGPLSLRGALSLEDQAQMAVEFKCRCYPGWQAPWCERKSM W |

**[TABLE 2]**

| Variant | Seque nce No. | Amino acid sequence |
|---|---|---|
| A132E | 2 | MAAHLLPICALFLTLLDMAQGFRGPLLPNRPFTTVWNANTQWC LERHGVDVDVSVFDVVANPGQTFRGPDMTIFYSSQLGTYPYYTP TGEPVFGGLPQNASLIAHLARTFQDILAAIPAPDFSGLAVIDWEE WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| A132N | 3 | MAAHLLPICALFLTLLDMAQGFRGPLLPNRPFTTVWNANTQWC LERHGVDVDVSVFDVVANPGQTFRGPDMTIFYSSQLGTYPYYTP TGEPVFGGLPQNASLIAHLARTFQDILAAIPAPDFSGLAVIDWEN WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI |
| | | QLTPGGGPLSLRGALSLEDQAQMAVEFKCRCYPGWQAPWCERK SMW |
| A132D | 4 | MAAHLLPICALFLTLLDMAQGFRGPLLPNRPFTTVWNANTQWC LERHGVDVDVSVFDVVANPGQTFRGPDMTIFYSSQLGTYPYYTP TGEPVFGGLPQNASLIAHLARTFQDILAAIPAPDFSGLAVIDWED WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| A132Y | 5 | MAAHLLPICALFLTLLDMAQGFRGPLLPNRPFTTVWNANTQWC LERHGVDVDVSVFDVVANPGQTFRGPDMTIFYSSQLGTYPYYTP TGEPVFGGLPQNASLIAHLARTFQDILAAIPAPDFSGLAVIDWEY WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| A132S | 6 | MAAHLLPICALFLTLLDMAQGFRGPLLPNRPFTTVWNANTQWC LERHGVDVDVSVFDVVANPGQTFRGPDMTIFYSSQLGTYPYYTP TGEPVFGGLPQNASLIAHLARTFQDILAAIPAPDFSGLAVIDWES WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| A132H | 7 | MAAHLLPICALFLTLLDMAQGFRGPLLPNRPFTTVWNANTQWC LERHGVDVDVSVFDVVANPGQTFRGPDMTIFYSSQLGTYPYYTP TGEPVFGGLPQNASLIAHLARTFQDILAAIPAPDFSGLAVIDWEH WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| A132G | 8 | MAAHLLPICALFLTLLDMAQGFRGPLLPNRPFTTVWNANTQWC LERHGVDVDVSVFDVVANPGQTFRGPDMTIFYSSQLGTYPYYTP TGEPVFGGLPQNASLIAHLARTFQDILAAIPAPDFSGLAVIDWEG WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |

**[TABLE 3]**

| Variant | Seque nce No. | Amino acid sequence |
|---|---|---|
| S76D | 9 | MAAHLLPICALFLTLLDMAQGFRGPLLPNRPFTTVWNANTQWC LERHGVDVDVSVFDVVANPGQTFRGPDMTIFYDSQLGTYPYYTP TGEPVFGGLPQNASLIAHLARTFQDILAAIPAPDFSGLAVIDWEA WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| S76E | 10 | MAAHLLPICALFLTLLDMAQGFRGPLLPNRPFTTVWNANTQWC LERHGVDVDVSVFDVVANPGQTFRGPDMTIFYESQLGTYPYYTP TGEPVFGGLPQNASLIAHLARTFQDILAAIPAPDFSGLAVIDWEA WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| S77D | 11 | MAAHLLPICALFLTLLDMAQGFRGPLLPNRPFTTVWNANTQWC LERHGVDVDVSVFDVVANPGQTFRGPDMTIFYSDQLGTYPYYTP TGEPVFGGLPQNASLIAHLARTFQDILAAIPAPDFSGLAVIDWEA WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI |
| | | QLTPGGGPLSLRGALSLEDQAQMAVEFKCRCYPGWQAPWCERK SMW |
| S77E | 12 | MAAHLLPICALFLTLLDMAQGFRGPLLPNRPFTTVWNANTQWC LERHGVDVDVSVFDVVANPGQTFRGPDMTIFYSEQLGTYPYYTP TGEPVFGGLPQNASLIAHLARTFQDILAAIPAPDFSGLAVIDWEA WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| Y85D | 13 | MAAHLLPICALFLTLLDMAQGFRGPLLPNRPFTTVWNANTQWC LERHGVDVDVSVFDVVANPGQTFRGPDMTIFYSSQLGTYPYDTP TGEPVFGGLPQNASLIAHLARTFQDILAAIPAPDFSGLAVIDWEA WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| T86D | 14 | MAAHLLPICALFLTLLDMAQGFRGPLLPNRPFTTVWNANTQWC LERHGVDVDVSVFDVVANPGQTFRGPDMTIFYSSQLGTYPYYDP TGEPVFGGLPQNASLIAHLARTFQDILAAIPAPDFSGLAVIDWEA WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| T86E | 15 | MAAHLLPICALFLTLLDMAQGFRGPLLPNRPFTTVWNANTQWC LERHGVDVDVSVFDVVANPGQTFRGPDMTIFYSSQLGTYPYYEP TGEPVFGGLPQNASLIAHLARTFQDILAAIPAPDFSGLAVIDWEA WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| P87D | 16 | MAAHLLPICALFLTLLDMAQGFRGPLLPNRPFTTVWNANTQWC LERHGVDVDVSVFDVVANPGQTFRGPDMTIFYSSQLGTYPYYTD TGEPVFGGLPQNASLIAHLARTFQDILAAIPAPDFSGLAVIDWEA WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| P87E | 17 | MAAHLLPICALFLTLLDMAQGFRGPLLPNRPFTTVWNANTQWC LERHGVDVDVSVFDVVANPGQTFRGPDMTIFYSSQLGTYPYYTE TGEPVFGGLPQNASLIAHLARTFQDILAAIPAPDFSGLAVIDWEA WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| T88D | 18 | MAAHLLPICALFLTLLDMAQGFRGPLLPNRPFTTVWNANTQWC LERHGVDVDVSVFDVVANPGQTFRGPDMTIFYSSQLGTYPYYTP DGEPVFGGLPQNASLIAHLARTFQDILAAIPAPDFSGLAVIDWEA WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| G89D | 19 | MAAHLLPICALFLTLLDMAQGFRGPLLPNRPFTTVWNANTQWC LERHGVDVDVSVFDVVANPGQTFRGPDMTIFYSSQLGTYPYYTP TDEPVFGGLPQNASLIAHLARTFQDILAAIPAPDFSGLAVIDWEA WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |

**[TABLE 4]**

| Variant | Seque nce No. | Amino acid sequence |
|---|---|---|
| S77D/A 132E | 20 | MAAHLLPICALFLTLLDMAQGFRGPLLPNRPFTTVWNANTQWC LERHGVDVDVSVFDVVANPGQTFRGPDMTIFYSDQLGTYPYYTP TGEPVFGGLPQNASLIAHLARTFQDILAAIPAPDFSGLAVIDWEE WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| S77E/A 132E | 21 | MAAHLLPICALFLTLLDMAQGFRGPLLPNRPFTTVWNANTQWC LERHGVDVDVSVFDVVANPGQTFRGPDMTIFYSEQLGTYPYYTP TGEPVFGGLPQNASLIAHLARTFQDILAAIPAPDFSGLAVIDWEE WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| S77D/T 86D | 22 | MAAHLLPICALFLTLLDMAQGFRGPLLPNRPFTTVWNANTQWC LERHGVDVDVSVFDVVANPGQTFRGPDMTIFYSDQLGTYPYYD PTGEPVFGGLPQNASLIAHLARTFQDILAAIPAPDFSGLAVIDWE AWRPRWAFNWDTKDIYRQRSRALVQAQHPDWPAPQVEAVAQ DQFQGAARAWMAGTLQLGRALRPRGLWGFYGFPDCYNYDFLS PNYTGQCPSGIRAQNDQLGWLWGQSRALYPSIYMPAVLEGTGK SQMYVQHRVAEAFRVAVAAGDPNLPVLPYVQIFYDTTNHFLPL DELEHSLGESAAQGAAGVVLWVSWENTRTKESCQAIKEYMDTT LGPFILNVTSGALLCSQALCSGHGRCVRRTSHPKALLLLNPASFSI QLTPGGGPLSLRGALSLEDQAQMAVEFKCRCYPGWQAPWCERK SMW |
| S77D/T 86E | 23 | MAAHLLPICALFLTLLDMAQGFRGPLLPNRPFTTVWNANTQWC LERHGVDVDVSVFDVVANPGQTFRGPDMTIFYSDQLGTYPYYE PTGEPVFGGLPQNASLIAHLARTFQDILAAIPAPDFSGLAVIDWE AWRPRWAFNWDTKDIYRQRSRALVQAQHPDWPAPQVEAVAQ DQFQGAARAWMAGTLQLGRALRPRGLWGFYGFPDCYNYDFLS PNYTGQCPSGIRAQNDQLGWLWGQSRALYPSIYMPAVLEGTGK SQMYVQHRVAEAFRVAVAAGDPNLPVLPYVQIFYDTTNHFLPL DELEHSLGESAAQGAAGVVLWVSWENTRTKESCQAIKEYMDTT LGPFILNVTSGALLCSQALCSGHGRCVRRTSHPKALLLLNPASFSI QLTPGGGPLSLRGALSLEDQAQMAVEFKCRCYPGWQAPWCERK SMW |
| S77D/T 86D/A1 32E | 24 | MAAHLLPICALFLTLLDMAQGFRGPLLPNRPFTTVWNANTQWC LERHGVDVDVSVFDVVANPGQTFRGPDMTIFYSDQLGTYPYYD PTGEPVFGGLPQNASLIAHLARTFQDILAAIPAPDFSGLAVIDWEE WRPRWAFNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |

**[TABLE 5]**

| Variant | Seque nce No. | Amino acid sequence |
|---|---|---|
| F139R | 25 | MAAHLLPICALFLTLLDMAQGFRGPLLPNRPFTTVWNANTQWC LERHGVDVDVSVFDVVANPGQTFRGPDMTIFYSSQLGTYPYYTP TGEPVFGGLPQNASLIAHLARTFQDILAAIPAPDFSGLAVIDWEA WRPRWARNWDTKDIYRQRSRALVQAQHPDWPAPQVEAVAQD QFQGAARAWMAGTLQLGRALRPRGLWGFYGFPDCYNYDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| Y210H | 26 | MAAHLLPICALFLTLLDMAQGFRGPLLPNRPFTTVWNANTQWC LERHGVDVDVSVFDVVANPGQTFRGPDMTIFYSSQLGTYPYYTP TGEPVFGGLPQNASLIAHLARTFQDILAAIPAPDFSGLAVIDWEA WRPRWAFNWDTKDIYRQRSRALVQAQHPDWPAPQVEAVAQD QFQGAARAWMAGTLQLGRALRPRGLWGFYGFPDCYNHDFLSP NYTGQCPSGIRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI QLTPGGGPLSLRGALSLEDQAQMAVEFKCRCYPGWQAPWCERK SMW |
| F139R/I 225D | 27 | [?] MAAHLLPICALFLTLLDMAQGFRGPLLPNRPFTTVWNANTQWC LERHGVDVDVSVFDVVANPGQTFRGPDMTIFYSSQLGTYPYYTP TGEPVFGGLPQNASLIAHLARTFQDILAAIPAPDFSGLAVIDWEA WRPRWARNWDTKDIYRQRSRALVQAQHPDWPAPQVEAVAQD QFQGAARAWMAGTLQLGRALRPRGLWGFYGFPDCYNYDFLSP NYTGQCPSGDRAQNDQLGWLWGQSRALYPSIYMPAVLEGTGKS QMYVQHRVAEAFRVAVAAGDPNLPVLPYVQIFYDTTNHFLPLD ELEHSLGESAAQGAAGVVLWVSWENTRTKESCQAIKEYMDTTL GPFILNVTSGALLCSQALCSGHGRCVRRTSHPKALLLLNPASFSI |
| | | QLTPGGGPLSLRGALSLEDQAQMAVEFKCRCYPGWQAPWCERK SMW |

These Hyal1 variants are capable of effectively degrading hyaluronic acid over a broad acidic-to-neutral pH range and exhibit higher enzymatic activity than wild-type Hyal1 at an acidic pH. Furthermore, since the Hyal1 variant according to an embodiment of the present invention is capable of degrading hyaluronic acid over an acidic to neutral pH range, it may effectively deliver drugs to various sites. This is because when hyaluronic acid in the extracellular matrix is hydrolyzed, the viscosity of hyaluronic acid decreases and permeability into tissues (skin) increases. In particular, since the subcutaneous tissue has a neutral pH of approximately 7.0 to 7.5, permeability may be increased by an embodiment of the present invention that is capable of degrading hyaluronic acid at neutral pH. Therefore, the drug delivery agent according to an embodiment of the present invention may include the variant according to an embodiment of the present invention, thereby effectively delivering drugs to various sites.

In this case, the drug is a component exhibiting pharmacological activity, and examples thereof may include an ocular relaxant, an anesthetic, an antibody therapeutic, or an anticancer agent, but are not limited thereto. The drug may form a composition together with the variant according to an embodiment of the present invention. That is, the variant according to an embodiment of the present invention may be in the form of a pharmaceutical composition, either alone or together with the drug.

The Hyal1 variant according to an embodiment of the present invention may be included in formulations applicable to various sites. For example, a formulation for subcutaneous administration, a formulation for intravenous injection, or an ophthalmic formulation according to an embodiment of the present invention may include the Hyal1 variant according to an embodiment of the present invention. In this case, the ophthalmic formulation may be an eye drop, and the eye drop may be used to promote diffusion of an anesthetic during ophthalmic surgery. In addition, the formulation for subcutaneous administration may specifically be a subcutaneous injection, and the subcutaneous injection may be used for hydrolysis of hyaluronic acid during a filler procedure. Furthermore, the formulation for intravenous injection may be used to increase the accessibility of an anticancer agent to tumor cells. This is because the formulation for intravenous injection may transport the Hyal1 variant according to an embodiment of the present invention through the bloodstream and hydrolyze hyaluronic acid overexpressed on the surface of tumor cells, thereby increasing the accessibility of the anticancer agent to tumor cells. In this way, the Hyal1 variant according to an embodiment of the present invention may be used for cosmetic purposes as well as therapeutic purposes. Accordingly, the formulation according to an embodiment of the present invention may be a pharmaceutical formulation and/or a cosmetic formulation.

The dosage of the Hyal1 variant according to an embodiment of the present invention is 10 ng/mL to 10 mg/mL per administration when administered by subcutaneous injection, preferably 10 ng/mL to 100 µg/mL. Administration may be carried out once daily or in several divided doses. Although the above dosage is based on an adult weighing 60 kg, it will be understood that the dosage may vary depending on body weight, physical condition, and other factors. The Hyal1 variant according to an embodiment of the present invention is primarily administered parenterally, for example, by subcutaneous injection, intravenous injection, or administration as eye drops.

The Hyal1 variant according to an embodiment of the present invention may also be formulated together with pharmaceutically acceptable additives into injections, eye drops, or transdermal patches.

The pharmaceutically acceptable additives may be selected and applied based on various factors well known to those skilled in the art, including but not limited to the specific bioactive substance employed, the concentration, stability, and intended bioavailability thereof; the disease or condition to be treated; the age, size, and general condition of a subject to be treated; and factors relating to routes of administration, such as nasal, oral, ocular, topical, transdermal, and intramuscular administration. Pharmaceutically acceptable additives commonly used for administering physiologically active substances by routes other than oral administration may include D5W (5% glucose in water), dextrose, and aqueous solutions containing physiological saline in an amount of up to 5% by volume. In the case of local injection, various injectable hydrogels may be used to enhance efficacy and prolong the duration of action. In addition, the pharmaceutically acceptable additives may include additional components capable of enhancing the stability of active ingredients, such as preservatives and antioxidants. The variant according to an embodiment of the present invention may be formulated by an appropriate method known in the art, and may be preferably formulated depending on the disease or condition to be treated or the components used therein.

The Hyal1 variant according to an embodiment of the present invention may be stored in physiological saline, and after addition of mannitol or sorbitol, may be lyophilized in an ampule. When used for administration, it may be dissolved in physiological saline or the like. In this case, the physiological saline may include a buffer, a stabilizer, and/or a surfactant.

In addition, the hyaluronic acid degradation and/or drug delivery method according to an embodiment of the present invention includes administering the Hyal1 variant according to an embodiment of the present invention to a mammal, including a human, in need of administration thereof. In this case, the administered Hyal1 variant according to an embodiment of the present invention may be an effective amount.

Furthermore, the use according to an embodiment of the present invention is the use of the Hyal1 variant according to an embodiment of the present invention for the manufacture of a hyaluronic acid degrading agent or a drug delivery agent.

The Hyal1 variant according to an embodiment of the present invention may be produced using genetic engineering techniques. For example, a fusion gene encoding a fusion protein composed of a fusion partner and the Hyal1 variant protein according to an embodiment of the present invention may be prepared through genetic engineering, and a host cell may be transformed with the fusion gene, followed by expression of the fusion protein in the host cell. Subsequently, the Hyal1 variant according to an embodiment of the present invention may be cleaved and isolated from the fusion protein using a protease or a chemical compound to produce the desired protein. Therefore, the Hyal1 variant according to an embodiment of the present invention may be effectively produced by the nucleic acid, expression vector, host cell, and/or method according to an embodiment of the present invention. Specifically, the nucleic acid according to an embodiment of the present invention encodes the variant according to an embodiment of the present invention. The nucleic acid may be present in a cell, in a cell lysate, or in a partially purified form or a substantially pure form. The nucleic acid may be, for example, DNA or RNA.

In addition, an expression vector according to an embodiment of the present invention may include the nucleic acid according to an embodiment of the present invention. In order to express the Hyal1 variant according to an embodiment of the present invention, DNA encoding the Hyal1 variant according to an embodiment of the present invention may be obtained by molecular biology techniques (e.g., PCR amplification or cDNA cloning using a hybridoma expressing the Hyal1 variant according to an embodiment of the present invention), and such DNA may be operably linked to transcription and translation control sequences and inserted into an expression vector, thereby producing the expression vector according to an embodiment of the present invention. In this case, "operably linked" may mean that a gene encoding the Hyal1 variant according to an embodiment of the present invention is ligated into the vector so that transcription and translation control sequences within the vector perform the intended function of regulating transcription and translation of the gene encoding the Hyal1 variant according to an embodiment of the present invention. The expression vector and expression control sequences are selected to be compatible with a host cell for expression. The gene encoding the Hyal1 variant according to an embodiment of the present invention is inserted into the expression vector by standard methods (e.g., ligation of complementary restriction enzyme sites on a gene fragment encoding the Hyal1 variant according to an embodiment of the present invention and on the vector, or blunt-end ligation when no restriction enzyme site is present). The recombinant expression vector includes regulatory sequences that control expression of the gene encoding the Hyal1 variant according to an embodiment of the present invention in a host cell. The "regulatory sequence" may include a promoter, an enhancer, and other expression control elements (e.g., a polyadenylation signal) that control transcription or translation of the gene encoding the Hyal1 variant according to an embodiment of the present invention. In this case, regulatory sequences may be selected differently depending on factors such as the choice of host cell to be transformed and the level of protein expression, and thus the design of the expression vector may vary.

In addition, the host cell according to an embodiment of the present invention may include the nucleic acid according to an embodiment of the present invention, or the expression vector according to an embodiment of the present invention. The host cell may be selected, for example, from the group consisting of animal cells, plant cells, yeast, *Escherichia coli*, and insect cells, but is not limited thereto.

Nucleic acids or expression vectors are introduced into a host cell by transformation or transfection. Various techniques commonly used for introducing exogenous nucleic acids (DNA or RNA) into prokaryotic or eukaryotic host cells for transformation or transfection may be employed, such as electroporation, calcium phosphate precipitation, DEAE-dextran transfection, or lipofection. Various expression host/vector combinations may be used to express the Hyal1 variant according to an embodiment of the present invention. Suitable expression vectors for eukaryotic hosts include, but are not limited to, expression control sequences derived from SV40, bovine papillomavirus, adenovirus, adeno-associated virus, cytomegalovirus, and retroviruses. Expression vectors that can be used in bacterial hosts include bacterial plasmids obtained from *Escherichia coli*, such as pET, pRSET, pBluescript, pGEX2T, pUC vectors, ColE1, pCR1, pBR322, pMB9 and derivatives thereof; plasmids having a wider host range, such as RP4; phage DNA, exemplified by numerous phage λ derivatives such as λgt10, λgt11, and NM989; and other DNA phages such as M13 and filamentous single-stranded DNA phages. Useful expression vectors for yeast cells include 2µm plasmids and derivatives thereof. Useful vectors for insect cells include pVL941.

In addition, a method for producing a Hyal1 variant according to an embodiment of the present invention may include culturing the host cell according to an embodiment of the present invention. When a recombinant expression vector capable of expressing the Hyal1 variant according to an embodiment of the present invention is introduced into a mammalian host cell, the variant according to an embodiment of the present invention may be produced by culturing the host cell for a period of time sufficient to allow expression of the Hyal1 variant in the host cell, preferably for a period of time sufficient to allow secretion of the Hyal1 variant according to an embodiment of the present invention into a culture medium in which the host cell is grown.

If necessary, the expressed Hyal1 variant according to an embodiment of the present invention may be isolated from the host cell and purified to be substantially pure. The isolation or purification of the Hyal1 variant according to an embodiment of the present invention may be performed using isolation or purification method commonly used for proteins, such as chromatography. Chromatography may include, for example, affinity chromatography, ion exchange chromatography, and hydrophobic interaction chromatography, or a combination therefore, but is not limited thereto. In addition to chromatography, filtration, ultrafiltration, salting-out, dialysis, and the like may also be used in combination.

Unless otherwise specified, the matters described for the Hyal1 variant, hyaluronic acid degrading agent, drug delivery agent, composition, use, formulation, and method according to embodiments of the present invention apply equally to one another insofar as they are not inconsistent with each other.

Hereinafter, the present invention will be described in more detail with reference to Examples, Comparative Examples, and Preparative Examples. However, the following Examples and Preparative Examples are provided only for illustrative purposes, and the scope of the present invention is not limited thereto.

The reagents used in the following Examples were commercially available products of the highest grade, and unless otherwise specified, were purchased from Sigma-Aldrich.

### <Example 1> Preparation of Hyal1 Variants

The human Hyal1 gene (Clone ID: hMU005315) was purchased from the Korea Human Gene Bank. The Hyal1 gene was amplified by PCR (Bioneer, AlllnOneCycler PCR system) using the pCMV-SPORT6-Hyal1 plasmid. The amplified gene was inserted into a vector designated pSGHV1 using the restriction enzymes XhoI and NotI, The pSGHV1 vector was constructed from the pSGHV0 vector by removing the human growth hormone (hGH) sequence and the TEV protease cleavage site. A DNA sequence encoding six His residues was positioned at the 3' end of the Hyall cDNA for protein purification using a Ni²⁺ column. Hyal1 variants were also constructed by PCR, and the DNA sequences of the resulting Hyal1 variants were confirmed by DNA sequencing. The primers used for PCR are listed in Table 6.

**[TABLE 6]**

| Hval1 | Sequence No. | Primer name | DNA sequence (5'-3') |
|---|---|---|---|
| WT | 28 | Hyall-F | CTCGAGGCCACCATGGCAGCCCACCTG CTTCC (XhoI site underlined) |
| | 29 | Hyal 1-R | GCGGCCGCTCAGTGGTGATGGTGATGA TGCCACATGC (NotI site underlined) |
| S76D | 30 | S76D-F | GACAATTTTCTATGACTCCCAG |
| | 31 | S76D-R | CCAGCTGGGAGTCATAGA |
| S76E | 32 | S76E-F | GACAATTTTCTATGAGTCCCAG |
| | 33 | S76E-R | CCAGCTGGGACTCATAGA |
| S77D | 34 | S77D-F | CAATTTTCTATAGCGACCAGCT |
| | 35 | S77D-R | AGCTGGTCGCTATAGAAAATTG |
| S77E | 36 | S77E-F | CTATAGCGAGCAGCTGGG |
| | 37 | S77E-R | CCCAGCTGCTCGCTAT |
| Y85D | 38 | Y85D-F | ACCTACCCCTACGACACGCCC |
| | 39 | Y85D-R | AGTGGGCGTGTCGTAGGGGTA |
| T86D | 40 | T86D-F | CTACCCCTACTACGATCCCAC |
| | 41 | T86D-R | CCCCAGTGGGATCGTAGTAG |
| T86E | 42 | T86E-F | CTACCCCTACTACGAGCCCAC |
| | 43 | T86E-R | CCCCAGTGGGCTCGTAGTAG |
| P87D | 44 | P87D-F | CCCTACTACACGGACACTGG |
| | 45 | P87D-R | CTCCCCAGTGTCCGTGTAG |
| P87E | 46 | P87E-F | CCCTACTACACGGAAACTGG |
| | 47 | P87E-R | GCTCCCCAGTTTCCGTG |
| G89D | 48 | G89D-F | TACACGCCCACTGATGAGCC |
| | 49 | G89D-R | CACAGGCTCATCAGTGGGC |
| A132E | 50 | A132E-F | TGGGAGGAATGGCGCCCAC |
| | 51 | A132E-R | GTGGGCGCCATTCCTCCCA |
| A132N | 52 | A132N-F | TGGGAGAACTGGCGCCCAC |
| | 53 | A132N-R | GTGGGCGCCAGTTCTCCCA |
| A132D | 54 | A132D-F | CATCGACTGGGAGGATTGGCG |
| | 55 | A132D-R | CGCCAATCCTCCCAGTCGATG |
| A132Y | 56 | A132Y-F | CATCGACTGGGAGTATTGGCGC |
| | 57 | A132Y-R | GCGCCAATACTCCCAGTCGATG |
| A132S | 58 | A132S-F | CATCGACTGGGAGAGTTGGCG |
| | 59 | A132S-R | CGCCAACTCTCCCAGTCGATG |
| A132H | 60 | A132H-F | CATCGACTGGGAGCATTGGCG |
| | 61 | A132H-R | CGCCAATGCTCCCAGTCGATG |
| A132G | 62 | A132G-F | CATCGACTGGGAGGGATGGCG |
| | 63 | A132G-R | CGTGGGCGCCATCCCTCC |
| F139R | 64 | F139R-F | CACGCTGGGCCAGAAACTGG |
| | 65 | F139R-R | GGTGTCCCAGTTTCTGGCCCA |
| Y210H | 66 | Y210H-F | CCTGACTGCTACAACCATGAC |
| | 67 | Y210H-R | GCTTAGAAAGTCATGGTTGTAGC |
| I225D | 68 | I225D-F | GCCCATCAGGCGACCGTGC |
| | 69 | I225D-R | GTCATTTTGGGCACGGTCGCCTG |

### <Example 2> Confirmation of Enzymatic Activity and Expression of Hyal1 Ala132 Variants

CHO-K1 cells (purchased from the Korea Cell Line Bank) were transfected with plasmids containing the genes encoding the Hyal1 wild type or variants prepared in Example 1 using polyethylenimine (PEI, Sigma-Aldrich) or Lipofectamine 3000 (Thermo Fisher). When the CHO-K1 cells reached 90-95% confluence in a 6-well plate, 150 µL of DMEM medium containing 2 µg of plasmid DNA was mixed with 150 µL of DMEM medium containing 6 µg of PEI. After 30 min, the PEI-DNA mixture was transferred to the 6-well plate, and the cells were incubated in a 5% CO₂ incubator.

After 48 h of transfection, the cell culture medium was collected and centrifuged at 10,000 × g to obtain the supernatant. The enzymatic activities of the Hyal1 wild type and variants were measured using a substrate-gel assay. Specifically, electrophoresis was performed using a 10% SDS gel containing hyaluronic acid (1.0 mg/mL), after which SDS was removed incubating the gel in a buffer containing Triton X-100 (3% Triton X-100, 50 mM Tris, 100 mM NaCl, pH 7.5) at 4 °C for 2 h. Phosphate buffers containing 100 mM NaCl and adjusted to pH 4, 5, 6, and 7 using disodium phosphate and monosodium phosphate were prepared. Each SDS gel was placed in a phosphate buffer having a pH of 4 to 7, and the enzymatic reaction was performed to proceed for 4 to 17 h at 37 °C with shaking at 50 rpm. After completion of the enzymatic reaction, the hyaluronic acid in the SDS gel was stained with 1.0% Alcian blue. Because the purpose of the present invention was to investigate the enzymatic activity of Hyal1 variants under neutral pH conditions, pH 4 was used as a control, and enzymatic activity at pH 6 and pH 7 was examined.

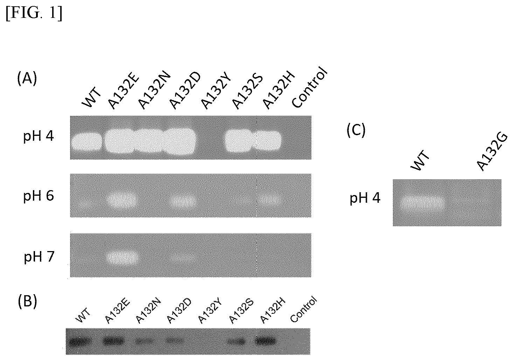

FIG. 1(A) illustrates the results of an experiment examining whether the Hyal1 variants exhibit enzymatic activity at different pH values. In the substrate-gel assay, hyaluronic acid hydrolyzed by the Hyal1 variants is not stained with Alcian blue and therefore appears as a white band. In FIG. 1(A), Control represents a control group transfected with the vector alone, WT represents wild-type Hyal1, and A132E, A132N, A132D, A132Y, A132S, and A132H represent the respective Hyal1 variants.

As shown in FIG. 1(A), at pH 4, all variants except A132Y hydrolyzed hyaluronic acid and exhibited enzymatic activity, whereas at pH 6, the A132E, A132D, and A132H variants exhibited enzymatic activity, and at pH 7, only the A132E and A132D variants exhibited enzymatic activity. Among these Hyal1 variants, A132E exhibited the highest enzymatic activity at pH 7. A132N and A132S, in which Ala132 was substituted with a polar amino acid, exhibited higher enzymatic activity than the wild type only at pH 4. A132H, in which Ala132 was substituted with His, exhibited enzymatic activity at pH 6 but not at pH 7.

In addition, to examine protein expression in CHO-K1 cells, Western blot analysis was performed after protein electrophoresis, as in the experiment shown in FIG. 1(A). Specifically, after transferring a 10% SDS gel to a nitrocellulose membrane at 100 V for 1 h, expression of the Hyal1 wild type and Ala132 variants was examined using a Hyal1 monoclonal antibody (1D10, Santa Cruz Biotechnology, Dallas, TX, USA) and an anti-mouse IgG binding protein (m-IgGκ BP-HRP, Santa Cruz Biotechnology). The results are shown in FIG. 1(B). FIG. 1(B) shows the results of an experiment conducted to confirm expression of Hyal1 variants. In FIG. 1(B), Control represents a control group transfected with the vector alone, WT represents wild-type Hyal1, and A132E, A132N, A132D, A132Y, A132S, and A132H represent the respective variants. As shown in FIG. 1(B), the WT, A132E, A132N, A132D, A132S, and A132H variant proteins were detected using the Hyal1 antibody, whereas the A132Y variant protein was not detected. From these results, it is inferred that the negative result observed for the A132Y variant in FIG. 1(A) was due to a lack of protein expression. FIG. 1(C) illustrates the results of an experiment comparing the enzymatic activities of wild-type Hyal1 and the A132G variant. The A132G variant showed reduced enzymatic activity than wild-type Hyal1.

The Hyal1 variant of the present invention hydrolyze hyaluronic acid via a general acid-base catalytic mechanism. The carboxyl group (-COOH) of the catalytic amino acid Glu131 serves as a proton donor to the hydroxyl leaving group, and thus protonation of Glu131 is important for catalysis. The results shown in FIG. 1(A) indicate that, when an amino acid adjacent to the catalytic amino acid Glu131 in the primary structure or tertiary structure is substituted with an acidic amino acid, the pKa value of Glu131 may increase due to electrostatic repulsion between negative charges, thereby enabling Glu131 to function as a proton donor over an acidic-to-neutral pH range. However, even among substitutions with acidic amino acid, A132E exhibited higher enzymatic activity than A132D at pH 7. Accordingly, these results indicate, particularly in the Hyal1 variant A132E according to an embodiment of the present invention, hyaluronic acid can be effectively degraded even under neutral pH conditions.

In addition, as shown in FIG. 1(A), the Ala132 variants exhibited higher enzymatic activity than wild-type Hyal1 at pH 4. These Ala132 variants share the common feature that the amino acid at position 132 is substituted with an amino acid having a larger size than Ala. In contrast, when Ala132 was substituted with Gly, which is smaller than Ala, the enzymatic activity of the A132G variant decreased, as shown in FIG. 1(C). Accordingly, these results indicate the Ala132 variants, excluding the Hyal1 A132G variant according to an embodiment of the present invention, can degrade hyaluronic acid more effectively than wild-type Hyal1 even under acidic pH conditions.

### <Example 3> Confirmation of Enzymatic Activity and Expression of Hyal1 Loop Variants

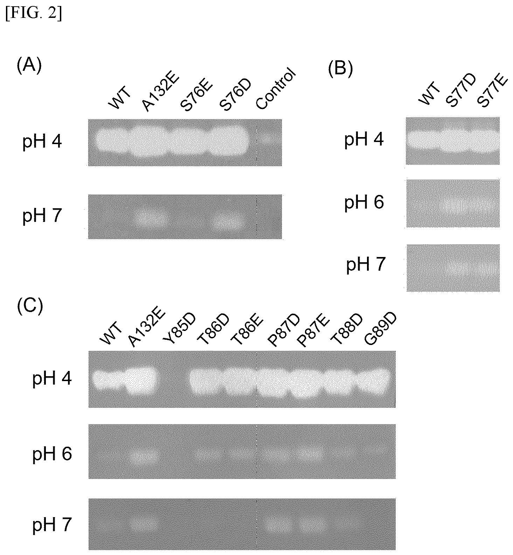

To investigate enzymatic activity of the constructed Hyal1 loop variants (S76D, S76E, S77D, S77E, Y85D, T86D, T86E, P87D, P87E, T88D, and G89D) over a pH range of 4 to 7, CHO-K1 cells were transfected with plasmids containing the genes encoding Hyal1 loop variants using the PEI or Lipofectamine method as described in Example 1. After 48 h of transfection, the cell culture medium was collected and centrifuged at 10,000 × g to obtain the supernatant. Using a substrate-gel assay, the enzymatic activities of Hyal1 WT and the loop variants present in the supernatant were examined by allowing the enzymatic reaction to proceed for 4 to 17 h at 37 °C in phosphate buffers having pH values ranging pH 4 to 7. The amount of hydrolyzed hyaluronic acid was then assessed using 1.0% Alcian blue staining. The results are shown in FIG. 2(A). FIG. 2(A) shows the results of an experiment conducted to determine whether wild-type Hyal1 and the loop variants exhibit enzymatic activity at different pH values. In FIG. 2(A), Control represents a control group transfected with the vector alone, WT represents wild-type Hyal1, and S76D, S76E, and A132E represent the respective variants. In FIG. 2(B), WT represents wild-type Hyal1, and S77D and S77E represent the respective variants. In FIG. 2(C), Y85D, T86D, T86E, P87D, P87E, T88D, G89D, and A132E represent the respective variants. In FIGS. 2(A) and 2(C), A132E was used as a positive control.

According to an embodiment of the present invention, the Hyal1 variants S76D, S77D, S77E, P87D, and P87E exhibited high enzymatic activity at pH 7. T86D exhibited low enzymatic activity at pH 7.

### <Example 4> Confirmation of Enzymatic Activity and Expression of Hyal1 Variants Having Combined Ala132 or Loop Amino Acid Substitutions

Among the Ala132 variants, the A132E variant, which exhibited the highest activity, was combined with one or more loop substitutions, and the enzymatic activities of the resulting variants were investigated over a pH range of 4 to 7. To investigate the enzymatic activities of the constructed Hyal1 variants (S77D/A132E, S77E/A132E, S77D/T86D, S77D/T86E, and S77D/T86D/A132E), CHO-K1 cells were transfected with plasmids containing the genes encoding these Hyal1 variants using the PEI or Lipofectamine method as described in Example 1. After 48 h of transfection, the cell culture medium was collected and centrifuged at 10,000 × g to obtain the supernatant. Using a substrate-gel assay, the enzymatic activities of the Hyal1 variants present in the supernatant were evaluated by allowing the enzyme reaction to proceed for 4 to 17 h at 37 °C in phosphate buffers having pH values from 4 to 7. The amount of hydrolyzed hyaluronic acid was then assessed using 1.0% Alcian blue staining.

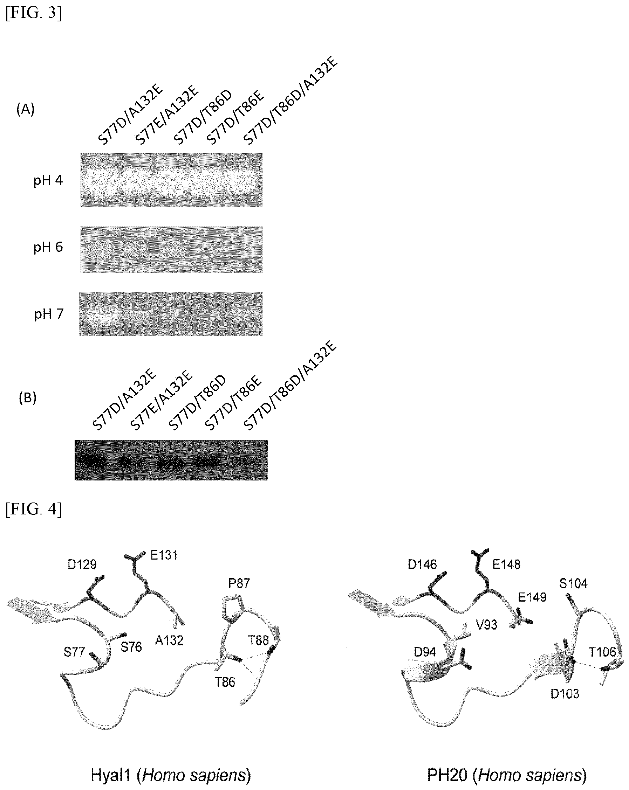

As shown in FIG. 3(A), the double or triple variants exhibited enzymatic activity by hydrolyzing hyaluronic acid not only at pH 4 but also at pH 7. In particular, differences in enzymatic activity were observed at pH 7, and the S77D/A132E variant exhibited the highest activity.

FIG. 3(B) shows the results of an experiment to confirm expression of the Hyal1 variants. In FIG. 3(B), the Hyal1 variants were successfully expressed in CHO-K1 cells.

### <Example 5> Comparison of Tertiary Structures of Human Hyal1 and PH20

To compare the positions of the mutated amino acids introduced into Hyal1 with the corresponding amino acids in PH20, tertiary structures of human Hyal1 and PH20 were compared. A three-dimensional structural model of PH20 was constructed using Swiss-Model based on the crystal structure of Hyal1 (PDB code 2PE4). The structures of Hyal1 and PH20 were compared using the PyMOL program, and results are shown in FIG. 4. As shown in FIG. 4, Ser76, Ser77, Thr86, Pro87, Thr88, and Ala132 in Hyal1 are located adjacent to catalytic amino acid Glu131. In the three-dimensional structural model of PH20, Asp94, Asp103, and Glu149 are also located adjacent to catalytic amino acid Glu148.

Accordingly, in the Hyal1 variants of the present invention, in which amino acids are substituted with acidic amino acids, the pKa value of the catalytic amino acid Glu131 may be increased due to electrostatic repulsion between negative charged acidic amino acids and Glu131, thereby enabling effective degradation of hyaluronic acid even under neutral pH conditions.

In addition, the Hyal1 variants of the present invention appear to exhibit increased enzymatic activity under acidic pH conditions when an amino acid larger than Ala is introduced at position Ala132, thereby increasing destabilizing the loop containing the catalytic amino acid Glu131 and increasing its structural flexibility.

### <Example 6> Confirmation of Enzymatic Activity and Expression of F139R and Y210H Variants

Another approach for increasing the pKa value of the catalytic amino acid Glu131 in Hyal1 is to substitute an amino acid adjacent to Glu131 with a basic amino acid, thereby decreasing the pKa value of an acidic or polar amino acid that forms an ionic bond with the basic amino acid. In the tertiary structure of Hyal1, Phe139, Asp206, Ile225, and Tyr210 are located adjacent to the catalytic amino acid Glu131. When the hydrophobic amino acid Phe139 is substituted with Arg, the pKa value of Tyr210 may decrease due to an ionic bond between Arg139 and Tyr210, which may in turn increase the pKa value of catalytic amino acid Glu131. In addition, when the hydrophobic amino acids Phe139 and Ile225 are substituted with Arg and Asp, respectively, the positive charge of Arg139 may be strengthened through an ionic interaction between Arg139 and Asp225. As a result, deprotonation of the hydroxyl group of Tyr210 through the ionic interaction between Arg139 and Tyr210 may be further promoted relative to the F139R single variant. Furthermore, when the polar amino acid Tyr210 is substituted with the basic amino acid His, the pKa value of Asp206 may decrease due to an ionic interaction between Asp206 and His210, thereby increasing the pKa value of the catalytic amino acid Glu131.

To investigate the enzymatic activities of the constructed Hyal1 variants (F139R, F139R/I225D, and Y210H) over a pH range of 4 to 7, CHO-K1 cells were transfected with plasmids containing the genes encoding these Hyal1 variants using the PEI or Lipofectamine method as described in Example 1. After 48 h of transfection, the cell culture medium was collected and centrifuged at 10,000 × g to obtain the supernatant. Using a substrate-gel assay, the enzymatic activities of Hyal1 WT and the variants present in the supernatant were evaluated by allowing the enzyme reaction to proceed for 4 to 17 h at 37 °C in phosphate buffers having pH values from 4 to 7. The amount of hydrolyzed hyaluronic acid was then assessed using 1.0% Alcian blue staining.

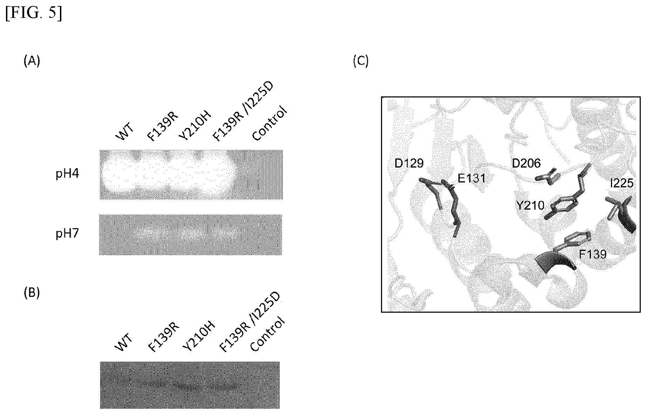

In FIG. 5(A), Control represents a control group transfected with the vector alone, WT represents wild-type Hyal1, and F139R, Y210H, and F139R/I225D represent the respective Hyal1 variants. While WT exhibited enzymatic activity only at pH 4, the F139R, Y210H, and F139R/I225D variants exhibited enzymatic activity at both pH 4 and pH 7.

FIG. 5(B) shows the results of an experiment conducted to confirm expression of the Hyal1 variants. In FIG. 5(B), the Hyal1 variants were successfully expressed in CHO-K1 cells.

FIG. 5(C) shows the tertiary structure of Hyal1 with the above described amino acids indicated therein. Asp206 and Tyr210 are located adjacent to the catalytic amino acid Glu131.

Based on the above results, the Hyal1 variants of the present invention can effectively degrade hyaluronic acid not only under acidic pH conditions but also under neutral pH conditions, thereby providing enhanced utility. In other words, because the Hyal1 variants of the present invention are capable of degrading hyaluronic acid even at a neutral pH, they may be used for drug delivery and in various formulations or applications, including formulations for subcutaneous administration, formulations for intravenous injection, and ophthalmic formulations.

Furthermore, based on the above results, the Hyal1 variants can be effectively produced using the nucleic acid, expression vector, host cell, and method of the present invention.

### <Preparative Example 1> Preparation of Liquid Formulation

One µg of the Hyal1 variant protein prepared in Examples 2 to 6 was dissolved in PBS to prepare 1 mL of a solution. The resulting solution was filled into an ampule for injection or a sterile container to prepare an injection solution or an ophthalmic formulation. The formulations thus prepared may be used as a hyaluronic acid degrading agent, a drug delivery agent, or the like. In particular, the injection formulation may be used as a formulation for subcutaneous administration or a formulation for intravenous injection, and the ophthalmic formulation may be used as an ophthalmic preparation.

### [Sequence Listing]

Sequence listing electronic file attached.

## Claims

1. A hyaluronidase Hyal1 variant exhibiting enzymatic activity at a neutral pH,
wherein, based on human hyaluronidase Hyal1 comprising Glu 131 as a catalytic amino acid in SEQ ID NO: 1,
one or more amino acids adjacent to the catalytic amino acid Glu 131 in the primary structure or tertiary structure are substituted with amino acids that increase a pKa value of Glu 131.

2. The hyaluronidase Hyal1 variant exhibiting enzymatic activity at a neutral pH according to claim 1, wherein the amino acid substitution comprises one or more substitutions selected from S76D and P87D.

3. A hyaluronidase Hyal1 variant having increased enzymatic activity at an acidic pH,
wherein, based on human hyaluronidase Hyal1 comprising Glu 131 as a catalytic amino acid in SEQ ID NO: 1,
one or more amino acids adjacent to Glu 131 in the primary structure or tertiary structure are substituted.

4. The hyaluronidase Hyal1 variant having increased enzymatic activity at an acidic pH according to claim 3, wherein the amino acid substitution comprises substituting one or more amino acids adjacent to Glu 131 in the primary structure or tertiary structure with an acidic amino acid.

5. The hyaluronidase Hyal1 variant having increased enzymatic activity at an acidic pH according to claim 4, wherein the amino acid substitution comprises substituting one or more amino acids located at positions 76, 77, 86, 87, 88, and 89 of SEQ ID NO: 1 with an acidic amino acid.

6. The hyaluronidase Hyal1 variant having increased enzymatic activity at an acidic pH according to claim 5, wherein the amino acid substitution comprises one or more substitutions selected from S76D, S77D, S77E, T86D, T86E, P87D, P87E, T88D, and G89D.

7. The hyaluronidase Hyal1 variant having increased enzymatic activity at an acidic pH according to claim 3, wherein an amino acid at position 132 adjacent to Glu 131 is substituted with an amino acid larger than alanine.

8. The hyaluronidase Hyal1 variant having increased enzymatic activity at an acidic pH according to claim 7, wherein the amino acid substitution comprises one or more substitutions selected from A132E, A132N, A132D, A132S, and A132H.

9. A nucleic acid encoding the hyaluronidase Hyal1 variant of claim 1.

10. A recombinant expression vector comprising the nucleic acid of claim 9.

11. A host cell transformed with the recombinant expression vector of claim 10.

12. A method for producing a hyaluronidase Hyal1 variant, comprising culturing the host cell of claim 10.

13. A hyaluronic acid degrading agent comprising the hyaluronidase Hyal1 variant of claim 1, 5, 7.

14. A drug delivery agent comprising the hyaluronidase Hyal1 variant of claim 1, 5, 7.

15. A formulation for subcutaneous administration comprising the hyaluronidase Hyal1 variant of claim 1.

16. A formulation for intravenous injection comprising the hyaluronidase Hyal1 variant of claim 1.

17. An ophthalmic formulation comprising the hyaluronidase Hyal1 variant of claim 1.
